(19) Europäisches Patentamt / European Patent Office / Office européen des brevets

(11) EP 2 985 041 B1

(12) EUROPEAN PATENT SPECIFICATION

(45) Date of publication and mention of the grant of the patent:
**19.07.2017 Bulletin 2017/29**

(21) Application number: **14782317.3**

(22) Date of filing: **10.04.2014**

(51) Int Cl.:
*A61L 27/50* (2006.01) *A61L 27/18* (2006.01)
*A61L 33/00* (2006.01) *A61L 27/54* (2006.01)

(86) International application number:
**PCT/JP2014/060376**

(87) International publication number:
**WO 2014/168197 (16.10.2014 Gazette 2014/42)**

(54) **ANTITHROMBOTIC ARTIFICIAL BLOOD VESSEL**

ANTITHROMBOTISCHES KÜNSTLICHES BLUTGEFÄSS

VAISSEAU SANGUIN ARTIFICIEL ANTITHROMBOTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2013 JP 2013084121**

(43) Date of publication of application:
**17.02.2016 Bulletin 2016/07**

(73) Proprietor: **Toray Industries, Inc.**
**Tokyo 103-8666 (JP)**

(72) Inventors:
- **TANAHASHI, Kazuhiro**
  **Otsu-shi**
  **Shiga 520-8558 (JP)**
- **SAKAGUCHI, Yuka**
  **Otsu-shi**
  **Shiga 520-8558 (JP)**
- **FUJITA, Masaki**
  **Otsu-shi**
  **Shiga 520-8558 (JP)**
- **KADOWAKI, Koji**
  **Otsu-shi**
  **Shiga 520-8558 (JP)**
- **TSUCHIKURA, Hiroshi**
  **Otsu-shi**
  **Shiga 520-2141 (JP)**
- **YAMADA, Satoshi**
  **Otsu-shi**
  **Shiga 520-2141 (JP)**

(74) Representative: **Kador & Partner**
**Corneliusstraße 15**
**80469 München (DE)**

(56) References cited:

**EP-A1- 0 128 741**
**EP-A1- 2 072 070**
**EP-A1- 2 518 100**
**EP-A2- 0 179 600**
**WO-A1-2008/032758**
**WO-A1-2011/078208**
**JP-A- H04 187 154**
**JP-A- 2004 525 888**
**JP-A- 2005 526 762**
**JP-A- 2007 252 893**
**JP-A- 2009 225 824**
**JP-A- 2011 245 282**
**JP-A- 2011 245 283**

- **DATABASE WPI Week 199404 Thomson Scientific, London, GB; AN 1994-032375 XP002761392, & JP H05 88611 B (TORAY IND INC) 22 December 1993 (1993-12-22)**
- **DATABASE WPI Week 198826 Thomson Scientific, London, GB; AN 1988-178821 XP002761393, & JP S63 115554 A (TORAY IND INC) 20 May 1988 (1988-05-20)**
- **DATABASE CA [Online] CHEMICAL ABSTRACTS SERVICE, COLUMBUS, OHIO, US; GENG, XUE ET AL: "Synthesis and characterization of anticoagulant heparinized .epsilon.-caprolactone/ Lactide copolymers and their electrospinning study", XP002761394, retrieved from STN Database accession no. 2010:198682 & GENG, XUE ET AL: "Synthesis and characterization of anticoagulant heparinized .epsilon.-caprolactone/ Lactide copolymers and their electrospinning study", GAODENG XUEXIAO HUAXUE XUEBAO , 31(1), 205-211 CODEN: KTHPDM; ISSN: 0251-0790, 2010,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE CA [Online] CHEMICAL ABSTRACTS SERVICE, COLUMBUS, OHIO, US; "Artificial blood vessels", XP002761501, retrieved from STN Database accession no. 1985:547188 & JP 6 077764 A 2 May 1985 (1985-05-02)
- DATABASE WPI Week 200967 Thomson Scientific, London, GB; AN 2009-P46213 XP002761502, & JP 2009 225824 A (TORAY IND INC) 8 October 2009 (2009-10-08)

## Description

### TECHNICAL FIELD

The present invention relates to an artificial blood vessel to be used for reconstruction, repair, or replacement of a blood vessel that has undergone damage and/or the like.

### BACKGROUND ART

The number of patients suffering from arteriosclerosis is increasing due to aging of population and an increase in the population with metabolic syndrome. Arteriosclerosis is abnormality of arterial walls. In arteriosclerosis, a hyperglycemic state or hyperlipidemic state of blood causes degeneration of the vascular wall, and, as a result, the vascular wall becomes weak or thickened, or calcification occurs to make the vascular wall hard and fragile. Although such blood vessel degeneration may occur at any site in the blood vessels in the body, peripheral blood vessels are especially remarkably affected by the degeneration.

Treatment of such a degenerated blood vessel is conventionally carried out by a minimally invasive endovascular treatment such as balloon dilation or stent placement using a catheter, or by surgery for replacement of the damaged blood vessel with a blood vessel of the patient himself or with an artificial blood vessel.

However, in cases where an artificial blood vessel is placed, the body recognizes the artificial blood vessel as a foreign substance, and blood clotting reaction proceeds on the blood-contacting surface of the artificial blood vessel to form a thrombus.

A blood vessel in the body has an intima having vascular endothelial cells on its surface contacting with blood, and the intima plays a role in inhibiting formation of a thrombus. Also in an indwelling artificial blood vessel, vascular endothelial cells cover the blood-contacting surface of the artificial blood vessel to form an intima. However, since the artificial blood vessel is recognized as a foreign substance until the intima is covered with the endothelial cells, means for preventing thrombus formation is required until the formation of the intima. In particular, at a site where an artificial blood vessel having a small diameter is used, the blood flow is low, so that deposition of thrombi easily occurs, and the blood vessel is likely to be clogged even with a small amount of thrombi. At present, the long-term performance of artificial blood vessels having small diameters is not good, and none of such artificial blood vessels is applicable for clinical use.

In order to solve these problems, development of artificial blood vessels has been conventionally carried out focusing on early intimal formation and early establishment of antithrombogenicity.

Examples of methods for promoting the intimal formation include a method in which a growth factor or an inducer of cells is carried by the artificial blood vessel, and a method in which an artificial blood vessel containing, as its constitutional material, a fabric, knit, or non-woven fabric of a fiber such as a polyester fiber is used. In particular, it is known that, in cases where an ultrafine fiber of less than 10 $\mu m$ is included, the size of the ultrafine fiber or the size of the fiber gap is suitable for cell growth or cell infiltration (Patent Documents 1, 2, and 3). It is also known that ultrafine fibers have effects to promote adhesion of platelets and to prevent leakage of blood from the blood vessel wall when the fibers are indwelling (Patent Document 4).

In a conventional method for giving antithrombogenicity to an artificial blood vessel, heparin is carried by the artificial blood vessel. Since the fiber itself does not have a capacity to carry heparin, the artificial blood vessel is made to carry a sufficient amount of heparin by a known method such as a method in which a gel composed of a biodegradable polymer or gelatin containing heparin is filled into fibers (Patent Document 5), or a method in which heparin is immobilized on the fiber surface by covalent bonds (Patent Document 6).

On the other hand, examples of known methods for giving antithrombogenicity using a substance other than heparin include methods in which an antithrombogenic agent or a polymer containing an antithrombogenic agent is bound to the surface using a high-energy ray such as $\gamma$-ray (Patent Documents 7 and 8).

### PRIOR ART DOCUMENTS

#### Patent Documents

Patent Document 1: JP 1875991 B
Patent Document 2: JP 1870688 B
Patent Document 3: JP 1338011 B
Patent Document 4: JP 4627978 B
Patent Document 5: JP 3799626 B

Patent Document 6: Japanese Translated PCT Patent Application Laid-open No. 2009-545333 published as JP 2014061408
Patent Document 7: WO 08/032758
Patent Document 8 WO 2011-078208

SUMMARY OF THE INVENTION

PROBLEMS TO BE SOLVED BY THE INVENTION

However, in cases where fiber gaps are filled such as the case of the artificial blood vessel described in Patent Document 5, cellular infiltration is prevented to cause a delay of intimal formation, and, furthermore, platelets adhere to gelatin and the like to rather promote thrombus formation, which is problematic. In cases where heparin is immobilized on the fiber surface by covalent bonds such as the case of the artificial blood vessel described in Patent Document 6, the amount of heparin that can be bound to the surface is limited because of the large molecular weight of heparin, and there is no long-term effect, which is problematic. Moreover, there is a problem that the cellular adhesiveness decreases since heparin, which has very high hydrophilicity, is bound to the fiber surface.

There are also methods, as described in Patent Documents 7 and 8, in which an antithrombogenic agent is bound to the surface by a high-energy ray such as $\gamma$-ray, but such methods have a problem that the antithrombogenic agent is degraded to cause a decrease in the activity, resulting in an insufficient antithrombotic performance. The reaction for the immobilization on the surface of the base material is achieved by $\gamma$-ray irradiation in a state where the antithrombogenic agent is adsorbed on the surface in an aqueous solution. However, an aqueous solution with high surface tension does not permeate into gaps of polyester ultrafine fibers having high hydrophobicity, and this results in unevenness of the surface treatment of the fiber surface in the inner portion, leading to induction of thrombogenic response in the portion that has not undergone the surface treatment. Moreover, since a hydrophilic polymer does not adhere to polyester, adherence of the antithrombogenic agent does not occur unless the antithrombogenic agent itself has affinity to polyester, so that the amount of the agent immobilized on the surface may decrease. Moreover, since the antithrombogenic agent is bound to the surface without intermediation by a hydrophilic polymer as a spacer, there is only a low degree of spatial freedom, and binding with thrombin is therefor limited, which is problematic.

Thus, conventional artificial blood vessels have failed in simultaneous achievement of cellular affinity and antithrombogenicity, and, in particular, there is no artificial blood vessel having a small diameter that is available for long-term clinical use at present in the world.

In view of this, an object of the present invention is to provide an artificial blood vessel which promotes intimal formation after indwelling, and is capable of maintaining antithrombogenicity during the intimal formation, and of maintaining its patency for a long time.

MEANS FOR SOLVING THE PROBLEMS

The present inventors intensively studied to solve the problems described above, and, as a result, discovered that, by covalent bonding of an antithrombogenic agent having a polymer chain other than heparin to an ultrafine fiber via the polymer chain by a condensation reaction, an addition reaction, or graft polymerization, antithrombogenicity can be given while the fine structure composed of the ultrafine fiber is maintained, that is, both cellular affinity and antithrombogenicity can be realized.

That is, the present invention provides the artificial blood vessels of the following (1) to (12).

(1) An artificial blood vessel which is a tubular fabric comprising, a fiber layer containing an ultrafine fiber(s) and an ultrafine fiber layer in the inside of the fiber layer, the ultrafine fiber layer being composed of an ultrafine fiber(s) having a fiber diameter(s) of not less than 10 nm and not more than 3 $\mu$m, wherein an antithrombogenic agent having a polymer chain other than heparin is covalently bound to the ultrafine fiber via the polymer chain, and the thrombin activity inhibition rate on the fiber surface at 37°C is not less than 60%.
(2) The artificial blood vessel according to (1), wherein the molecular weight of the antithrombogenic agent is not more than 3000.
(3) The artificial blood vessel according to (1) or (2), whose water permeability at 120 mmHg is not less than 100 mL/cm$^2$/min. and less than 4000 mL/cm$^2$/min.
(4) The artificial blood vessel according to any one of (1) to (3), wherein the thrombin activity inhibition rate in an extract obtained by 24 hours of extraction at 37°C in 10 mL of physiological saline per 1 g of the artificial blood vessel is less than 5%.
(5) The artificial blood vessel according to any one of (1) to (4), wherein the antithrombogenic agent has a guanidino group, guanido group, and/or amidino group

(6) The artificial blood vessel according to any one of (1) to (5), wherein the polymer chain is a polymer structure selected from polyalkylene glycol, polyvinyl alcohol, and polyvinyl pyrrolidone.

(7) The artificial blood vessel according to any one of (1) to (6), wherein the antithrombogenic agent is selected from the following Chemical Formulae (I) to (IV):

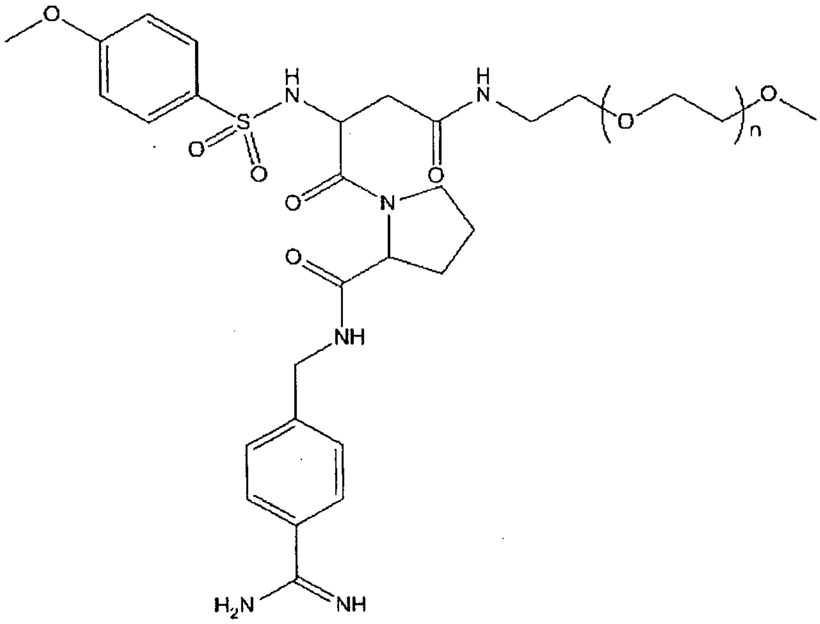

···(I);

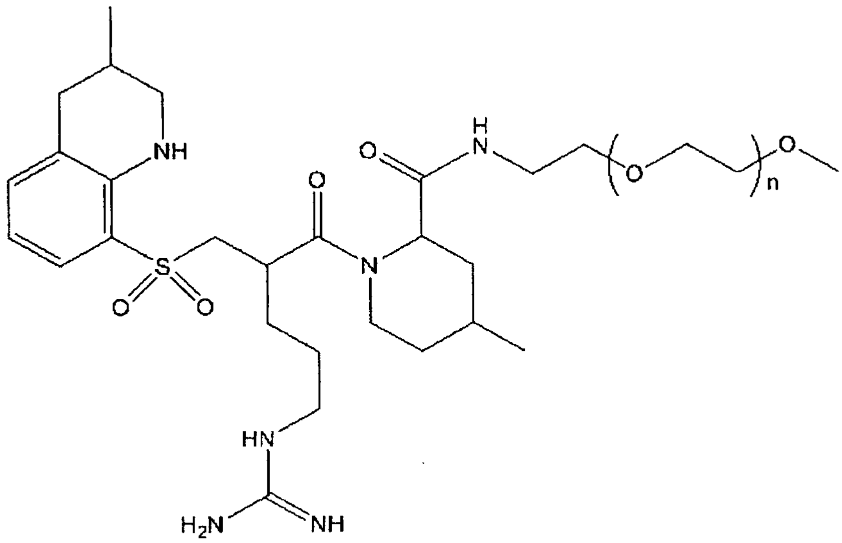

···(II);

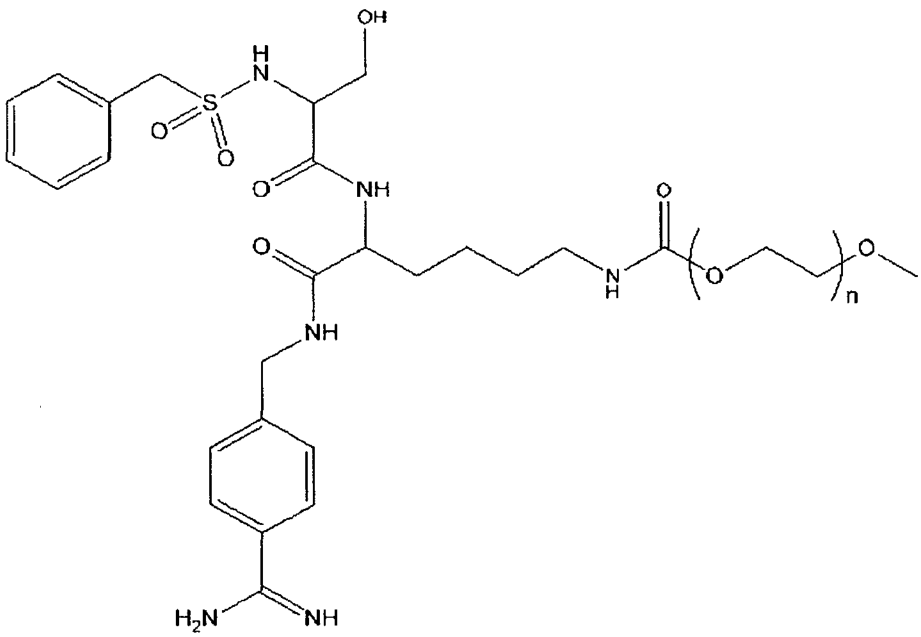

···(III);

and

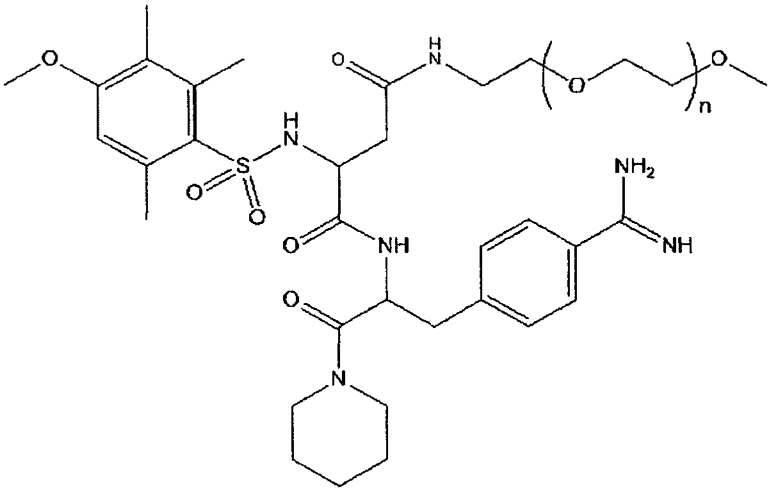

···(IV);

(wherein n represents an integer of 1 to 500).

(8) The artificial blood vessel according to any one of (1) to (7), wherein the fiber layer is composed of the ultrafine fiber(s) and a multifilament(s) having a total fineness of 1 to 60 decitex.

(9) The artificial blood vessel according to (8), wherein the fineness of single yarns constituting the multifilament is 0.5 to 10.0 decitex.

(10) The artificial blood vessel according to any one of (1) to (9), having a platelet adhesion rate of less than 20%.

(11) The artificial blood vessel according to any one of (1) to (10), wherein the tubular fabric is composed of a polyester fiber(s).

(12) The artificial blood vessel according to any one of (1) to (11), wherein the inner diameter of the tubular fabric is not less than 1 mm and less than 10 mm.

EFFECT OF THE INVENTION

By the present invention, an artificial blood vessel which promotes intimal formation after indwelling, and is capable of maintaining antithrombogenicity during the intimal formation, and of maintaining its patency for a long time can be provided.

BRIEF DESCRIPTION OF THE DRAWING

Fig. 1 is a schematic diagram showing the fiber structure of the artificial blood vessel of the present invention.

MODE FOR CARRYING OUT THE INVENTION

The ultrafine fiber in the present invention means a fiber having a fiber diameter of not less than 10 nm and not more than 3 μm. In cases where an artificial blood vessel having an ultrafine fiber is used, the number of scaffolds suitable for adhesion of living cells remarkably increases because of the extreme fineness of the fiber, and excellent cellular infiltration can be achieved. Favorable intimal formation occurs in an extremely early phase, and leakage of blood hardly occurs.

Since a strength that allows the artificial blood vessel to follow blood pressure and movement of tissues cannot be exerted with only ultrafine fibers, the artificial blood vessel of the present invention has a fiber structure composed of, as shown in Fig. 1, a fiber layer 2 in which ultrafine fibers 1 are dispersed in gaps of a basic tissue formed by a coarse texture, stitch, or the like constituted by thick fibers; and an ultrafine fiber layer 3 composed of ultrafine fibers 1, inside the fiber layer 2. The artificial blood vessel of the present invention is formed by making this fiber structure into a tubular shape.

As a production method for forming the fiber structure in which ultrafine fibers are dispersed in gaps of a basic tissue formed by a coarse texture, stitch, or the like, a common production method for ultrafine fibers may be employed. Together with a fiber having a size suitable for the strength of the basic tissue, a multicomponent fiber having a sea-island structure is subjected to weaving, knitting, or processing into a braid or a non-woven fabric, and then the sea structure of part of the multicomponent fiber is dissolved using an alkali or the like to perform ultrafining treatment. By this, the ultrafine fiber in the base fabric and the ultrafine fiber layer are preferably prepared.

Thereafter, a gap structure which is more desirable for cells can be achieved by interlacing the ultrafine fiber with the basic tissue by a water-jet process, air-jet process, or the like. In order to allow more effective exertion of the cellular affinity, formation of the ultrafine fiber layer on the blood-contacting surface can be further promoted by a method in which, for example, the blood-contacting surface is rubbed with a file to fuzz the surface.

The fiber material is not limited as long as it is a polymer having biocompatibility. Examples of the fiber material include polyester, polyethylene, polytetrafluoroethylene, polyurethane, polyamide, and nylon. Among these fiber materials, polyester, especially polyethylene terephthalate, is preferred since it has been conventionally clinically used as a material of artificial blood vessels, and has excellent strength.

The fiber may be in any form, and examples of the form include a spun yarn, multifilament yarn, monofilament yarn, and film split fiber yarn. From the viewpoint of strength, uniformity of physical properties, and flexibility, a multifilament yarn is excellent as the form of the fiber. The yarn may be either untwisted or twisted. The yarn may be crimped to a certain extent.

The total fineness of the fiber is preferably 1 to 60 decitex (Dtex), more preferably 1 to 40 decitex. The lower limit of the total fineness is more preferably 5 decitex, most preferably 10 decitex. The upper limit of the total fineness is more preferably 35 decitex, most preferably 25 decitex. With a total fineness of not less than 1 decitex, the strength required for the base fabric can be maintained, and, with a total fineness of not more than 40 decitex, the thickness of the base fabric can be reduced.

The single yarn fineness is preferably 0.5 to 10 decitex (Dtex), more preferably 0.5 to 3.0 decitex. The lower limit of the single yarn fineness is more preferably 1 decitex, and the upper limit of the single yarn fineness is more preferably 2 decitex. In cases where the single yarn fineness is not less than 3 decitex, the flexibility is deteriorated. In cases where the single yarn fineness is not more than 0.5 decitex, the hydrolysis rate is high, and there is a problem of deterioration of the strength.

In the tubular fabric which forms the artificial blood vessel, the cloth is provided as a fabric because of its excellent dimensional stability and strength.

For increasing the amount of the antithrombogenic agent immobilized on the fiber surface, ultrafine single-yarn-fineness multifilament yarns may be effectively placed in a part of the cloth. The single-yarn fiber diameter of this ultrafine single-yarn-fineness multifilament yarns is preferably 10 nm to 20 μm, more preferably 10 nm to 3 μm, most preferably 0.8 to 1.2 μm.

The size and the amount of the fiber gaps in the fiber layer and the ultrafine fiber layer of the artificial blood vessel can be represented using as an index the water permeability under a pressure of 120 mmHg, and the fiber gap is preferably 100 mL/cm$^2$/min. to 4000 mL/cm$^2$/min. For formation of an intima containing a stable vascular endothelial cell layer on the blood-contacting surface of the artificial blood vessel, a cell layer which supports the intima and mainly contains vascular smooth muscle and fibroblasts is important. Cells in this cell layer, together with vascular endothelial cells that migrate on the surface of the blood vessel, pass through fiber gaps, and infiltrate from the anastomotic site into the inside. Vascular endothelial cells not only infiltrate from the anastomotic site, but also infiltrate from sites on the

inner wall of the artificial blood vessel where openings are formed by blood capillaries that infiltrated from the outer wall of the artificial blood vessel through fiber gaps.

In view of this, the fiber gap is preferably not less than 100 $mL/cm^2/min$. since, in such cases, intimal formation due to infiltration of the inside of the fiber layer by cells and blood capillaries easily occurs. In cases where the fiber gap is not more than 4000 $mL/cm^2/min$., cellular pseudopodia more easily reach the inside of the fiber layer, and fill the gaps to prevent blood leakage, which is preferred.

The size of the artificial blood vessel of the present invention is not limited. The artificial blood vessel of the present invention is most effective as a thin artificial blood vessel having an inner diameter of not less than 1 mm and less than 10 mm.

The present invention realized both cellular affinity and antithrombogenicity since, in the present invention, an antithrombogenic agent having a polymer chain other than heparin is covalently bound, via the polymer chain, to the surface of the fiber and the ultrafine fiber constituting the basic tissue.

Among antithrombogenic agents having a polymer chain other than heparin, a low-molecular-weight antithrombogenic agent is preferably used. More specifically, an antithrombogenic agent having a molecular weight of not more than 3000 is preferred.

Since heparin, which is widely known as an antithrombogenic agent, is a large molecule having a molecular weight of 30,000 to 35,000 daltons, it can be immobilized on the surface in only a limited amount. Although low-molecular-weight heparins, whose molecular weights are lower than that of heparin, are also clinically used, even these low-molecular-weight heparins have molecular weights of as large as 4,000 to 6,000, which are about 10 times the molecular weights of synthetic antithrombogenic substances. Heparin can inhibit the activity of thrombin only after binding to antithrombin III and thrombin. Since the binding sites of antithrombin III and thrombin are separately present in the molecule, it is very difficult to control the immobilization on the surface while allowing these binding sites to be arranged in optimum positions. This difficulty also causes the low reaction efficiency of the immobilization on the surface. Moreover, the antithrombin activity itself of heparin is about 10 times lower than those of synthetic antithrombin substances. Thus, the activity of heparin is originally low. Moreover, it is known that there are, in the world, not a small number of patients with heparin-induced thrombocytopenia, who show excessive allergy to heparin. Heparin cannot be used completely freely when there is a social or ethical reason.

The antithrombin activity substance having a polymer chain used in the present invention is preferably a substance having a guanidino group, guanido group, and/or amidino group, more preferably a substance selected from the following General Formulae (I) to (IV):

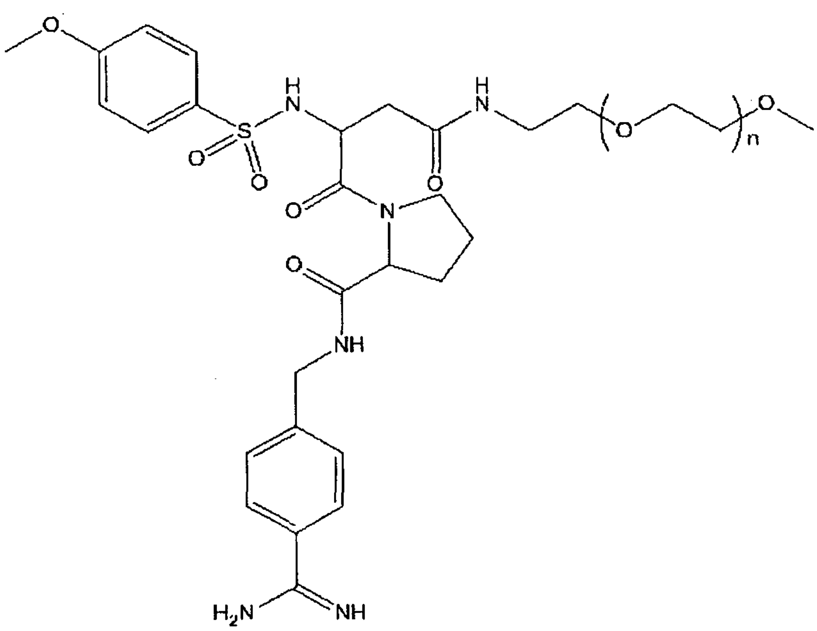

·(I);

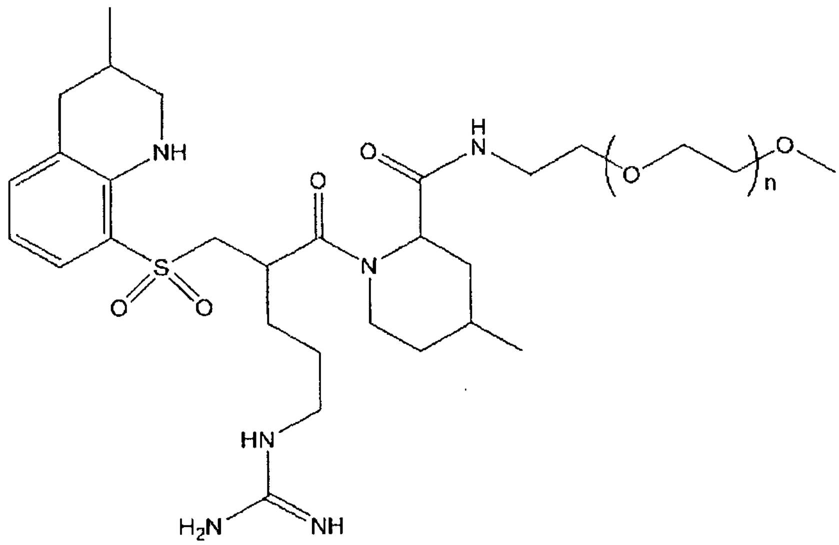

(II);

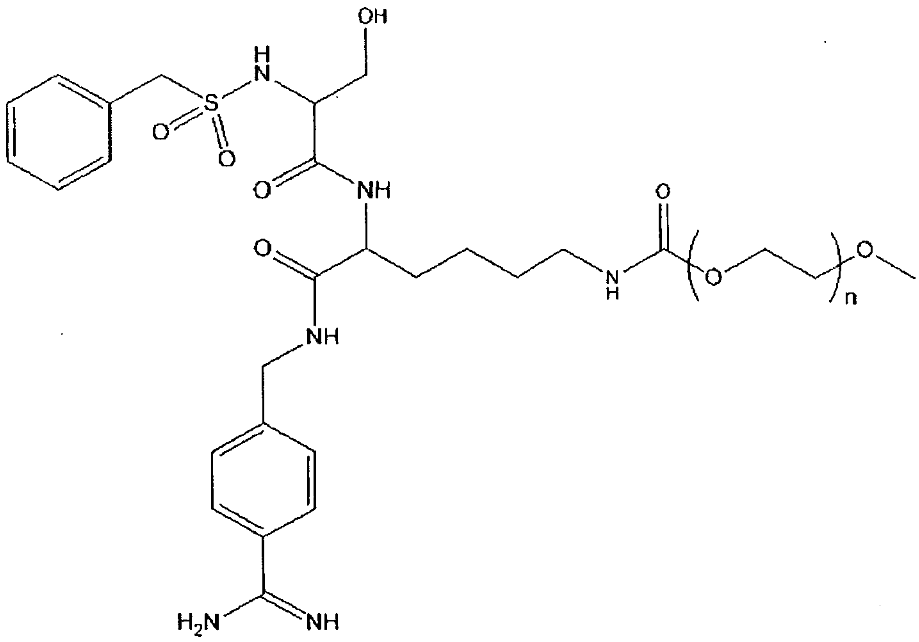

(III);

and

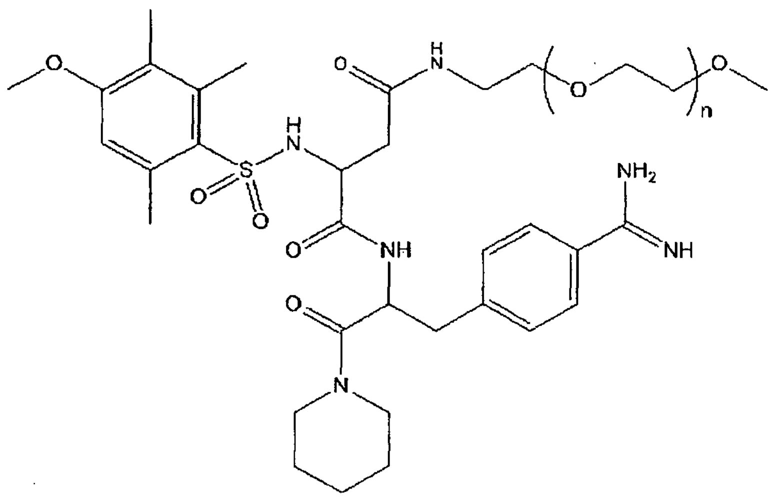

(IV).

Since these antithrombogenic agents having a polymer chain are immobilized on the fiber surface by covalent bonding via a functional group at the end of the polymer chain, elution of the agents does not occur, and the agents can maintain their effects on the surface for a long time. The method for immobilizing the antithrombogenic agent on the fiber surface by covalent bonding while maintaining its antithrombin activity is a method in which the reactive functional group to be used for the immobilization reaction is introduced to a site distant from the active site of the antithrombogenic agent, for example, to a site in the opposite side with respect to the polymer chain, to provide a derivative, and a chemical reaction such as condensation reaction, addition reaction, or graft polymerization is then performed to achieve the immobilization. Methods in which the antithrombogenic agent and the fiber, in their coexistence, are irradiated with a high-energy ray such as γ-ray or electron beam, and methods in which the fiber is subjected to plasma treatment and then brought into contact with the antithrombin agent, cannot be used since a highly reactive radical species deactivates the active site, or the orientation cannot be controlled such that the antithrombin agent can exert the maximum activity.

The immobilization of the antithrombogenic agent is preferably carried out by a method in which the distance between the immobilization site and the active site is as long as possible, and the reactive functional group is introduced via the polymer chain for securing the freedom after the immobilization. The polymer chain preferably has hydrophilicity. The structure of the hydrophilic polymer chain is not limited, and is preferably a high molecular structure selected from polyalkylene glycols such as polyethylene glycol (PEG), polypropylene glycol (PPG), and polyethylene glycol/polypropylene glycol copolymers (PEG-PPG); polyvinyl alcohol (PVA); and polyvinyl pyrrolidone (PVP); for exertion of the effect especially in blood, which is hydrophilic. The degree of polymerization of the hydrophilic polymer chain, n, is not limited, and is preferably 1 to 500 since, in cases where n is too large, the hydrophilicity increases, and therefore the cellular adhesiveness decreases.

The antithrombogenicity and the cellular affinity of the artificial blood vessel of the present invention are shown by measurement of the water permeability, the thrombin activity inhibition rate in an extract, the thrombin activity inhibition rate on the fiber surface, the platelet adhesion rate, the cell adhesion rate, and thrombus adhesion. These are measured by the following methods.

<Water Permeability>

Two sites are randomly sampled from the artificial blood vessel, and measurement is carried out twice for each sample by the method described below, followed by calculating the arithmetic mean of the measured values. The artificial blood vessel was cut along the axial direction, and a sample piece having a size of 1 cm × 1 cm was prepared. Between two doughnut-shaped packings with a diameter of 4 cm on each of which a hole having a diameter of 0.5 cm is formed by punching, the fabric sample having a size of 1 cm × 1 cm is sandwiched such that liquid flow is allowed only through the punched portion. The resultant is stored in a housing for a circular filtration filter. Water filtered through a reverse osmosis membrane is passed through this circular filtration filter at a temperature of 25°C for not less than 2 minutes until the sample piece sufficiently contains water. Under the conditions of a temperature of 25°C and a filtration differential pressure of 120 mmHg, external-pressure dead-end filtration of water filtered through a reverse osmosis membrane is carried out for 30 seconds to measure the amount of the water (mL) that permeates the portion with a diameter of 1 cm. The permeation volume is calculated by rounding the measured value to an integer. By converting the permeation volume (mL) to the value per unit time (min.) per effective area on the sample piece ($cm^2$), the water permeability at a pressure of 120 mmHg is determined.

In order to investigate the degree of elution of the antithrombogenic agent from the artificial blood vessel after the immobilization of the antithrombogenic agent, the thrombin activity inhibition rate in an extract may be measured by the following method. In terms of the thrombin activity inhibition rate in this extract, the thrombin activity inhibition rate in the extract at 37°C is preferably as low as possible. The thrombin activity inhibition rate is preferably less than 5%, more preferably less than 1%.

<Method for Measuring Thrombin Activity Inhibition Rate in Extract>

One gram of a ring-shaped sample prepared by cutting the artificial blood vessel into round slices in the transverse direction is cut into 10 small pieces each having a weight of 0.1 g along the longitudinal direction of the original blood vessel, and extraction is carried out with 10 mL of physiological saline per 1 g of the sample at 37°C for 24 hours. To 10 μL of sample-free physiological saline, or to 10 μL of the sample extract, 0.5 mL of 0.1 U/mL aqueous thrombin (Haematologic Technologies Inc.) solution and 0.5 mL of 200 μM aqueous S2238 (Sekisui Medical Co., Ltd.) solution are added. After leaving the resulting mixture to stand at 37°C for 45 minutes, the absorbance at 405 nm is measured using a microplate reader (Corona Electric Co., Ltd., MTP-300). Using the molar extinction coefficient of p-nitroaniline at a wavelength of 316 nm ($1.27 \times 10^4$ $mol^{-1} \cdot L \cdot cm^{-1}$), the amount of S2238 degraded per unit time, that is, the degradation rate of S2238, was calculated. As shown in Equation 1 below, the ratio of the degradation rate in the extract to the degradation rate in the sample-free physiological saline, which is taken as 100, is determined, to calculate the thrombin activity inhibition rate at 37°C. The Equation 1 is used to provide the thrombin activity inhibition rate in the extract.

$$\text{Thrombin activity inhibition rate (\%)} = (1 - \text{degradation rate in extract} / \text{degradation rate in physiological saline}) \times 100 \ldots \text{(Equation 1)}$$

In the artificial blood vessel after the immobilization of the antithrombogenic agent, the antithrombin performance on the fiber surface can be measured by the following method for measuring the thrombin activity inhibition rate on the fiber surface. In terms of the antithrombin activity inhibition rate on the fiber surface, the thrombin activity inhibition rate is preferably as high as possible. The thrombin activity inhibition rate is preferably not less than 60%, more preferably not less than 80%.

<Method for Measuring Thrombin Activity Inhibition Rate on Fiber Surface>

One gram of a ring-shaped sample prepared by cutting the artificial blood vessel into round slices in the transverse direction is cut into 10 small pieces each having a weight of 0.1 g along the longitudinal direction of the original blood vessel, and 5 mL of 0.1 U/mL aqueous thrombin (Haematologic Technologies Inc.) solution and 0.5 mL of 200 μM aqueous S2238 (Sekisui Medical Co., Ltd.) solution per 1 g of the sample are added thereto. After leaving the resulting mixture to stand at 37°C for 45 minutes, the absorbance at 405 nm is measured using a microplate reader (Corona Electric Co., Ltd., MTP-300). Using the molar extinction coefficient of p-nitroaniline at a wavelength of 316 nm ($1.27 \times 10^4$ $mol^{-1} \cdot L \cdot cm^{-1}$), the amount of S2238 degraded per unit time, that is, the degradation rate of S2238, was calculated. Based on this degradation rate, as shown in Equation 2 below, the ratio of the degradation rate on the fiber surface to the degradation rate in the sample-free physiological saline, which is taken as 100, is determined, to calculate the thrombin activity inhibition rate at 37°C. The Equation 2 is used to provide the thrombin activity inhibition rate on the fiber surface.

$$\text{Thrombin activity inhibition rate (\%)} = (1 - \text{degradation rate on fiber surface} / \text{degradation rate in physiological saline}) \times 100 \ldots \text{(Equation 2)}$$

In the artificial blood vessel after the immobilization of the antithrombogenic agent, the platelet adhesion rate on the fiber surface can be measured by the following method for measuring the platelet adhesion rate on the fiber surface. The lower the platelet adhesion rate on the fiber surface, the better. The platelet adhesion rate on the fiber surface is preferably less than 20%.

<Method for Measuring Platelet Adhesion Rate on Fiber Surface>

The artificial blood vessel is cut along the axial direction, and a disk sample with a diameter of 12 mm is prepared

by punching using a puncher. The sample piece is placed in a well of a 24-well microplate for cell culture (Sumitomo Bakelite Co., Ltd.) such that the blood-contacting surface faces upward, and a metallic pipe-shaped weight with a wall thickness of 3 mm is loaded thereon. Platelet-rich plasma prepared separately is added to the well such that the number of platelets is about $10^8$ per well. The microplate is left to stand at 37°C for 2 hours, and the sample is then removed therefrom and rinsed with PBS(-) (Nissui), followed by destroying platelets and measuring the activity of generated LDH according to the protocol described for LDH Cytotoxicity Detection kit (Takara Bio Inc.). Based on a calibration curve prepared separately, the number of adherent platelets is determined. As shown in Equation 3 below, the ratio of the number of platelets after the contact with the sample piece to the number of platelets in the platelet-rich plasma before the contact is determined, to provide the platelet adhesion rate.

$$\text{Platelet adhesion rate (\%)} = (\text{number of adherent platelets after the contact} / \text{number of platelets in platelet-rich plasma}) \times 100 \ldots \text{(Equation 3)}$$

<Cellular Adhesiveness>

The artificial blood vessel is cut along the axial direction, and a disk sample with a diameter of 12 mm is prepared by punching using a puncher. The sample piece is placed in a well of a 24-well microplate for cell culture (Sumitomo Bakelite Co., Ltd.), and a metallic pipe-shaped weight with a wall thickness of 3 mm is loaded thereon. Normal human umbilical vein endothelial cells (Takara Bio Inc.) suspended in DMEM medium supplemented with 10% FCS are added thereto such that $10^6$ cells are contained in the well. The microplate is left to stand at 37°C for 12 hours, and the sample is then removed therefrom and rinsed with PBS(-) (Nissui), followed by detaching the cells by enzyme treatment and measuring the number of detached cells using an MTT Assay Kit (Funakoshi Corporation). As shown in Equation 4 below, the ratio of the number of adherent cells to the number of cells plated on the sample is determined, to provide the cell adhesion rate.

$$\text{Cell adhesion rate (\%)} = (\text{number of adherent cells} / \text{number of cells plated}) \times 100 \ldots \text{(Equation 4)}$$

<Thrombus Adhesion in Blood Circulation>

The artificial blood vessel was cut into a length of 4 cm, and connected to a polyvinyl chloride tube having the same inner diameter as the artificial blood vessel, and a length of 32 cm. Into the tube, 4.5 mL of human fresh blood supplemented with heparin at a final concentration of 0.5 IU/mL was introduced, and both ends were immediately sealed to form a loop. The prepared loop was fixed on a frame attached to a rotor operated at a rotation speed of 14 rpm in a thermo-hygrostat drier whose temperature was preliminarily adjusted to 37°C, and rotated for 120 minutes. The loop is then taken out, and the polyvinyl chloride tube is cut to remove blood, followed by rinsing with PBS(-) (Nissui). Thereafter, the presence or absence of thrombi formed in the artificial blood vessel is quantified. The test is carried out with N=3. The same test is carried out using, as a negative control, PBS(-) instead of the human fresh blood. The dry weight of the artificial blood vessel with a length of 4 cm is measured before the test and after the removal of blood and rinsing, and the difference between these measured values is regarded as the thrombus weight, and its mean and standard deviation are calculated. In cases where the mean for the sample is not less than (mean + 3 × standard deviation) for the negative control, the result is evaluated as "+", and, in cases where the mean for the sample is less than this value, the result is evaluated as "-". In cases where leakage of blood is found through the artificial blood vessel during the circulation, the result is evaluated as "leakage" irrespective of the amount of blood leaked, and the test is stopped.

EXAMPLES

Examples of the artificial blood vessel of the present invention are concretely described below in detail.

(Examples)

A tubular fabric was prepared as a plain-weave tissue using polyethylene terephthalate of 55 Dtex-48 f as a warp, and polymer array fiber of 245 Dtex-40 f as a weft. The polymer array fiber used therefor was composed of 20 parts of polystyrene as sea-component and 80 parts of polyethylene terephthalate as island-component, and the number

of islands was 36/f. This tubular fabric was sufficiently treated with an aqueous sodium hydroxide solution at 80°C, and immersed in toluene. Subsequently, the fabric was subjected to raising by using a raising machine, and then to water-jet punching.

The tubular fabric after the treatments described above was treated with 0.5% aqueous sodium hydroxide solution, and then subjected to oxidation treatment with 5% potassium permanganate. Subsequently, the tubular fabric was immersed in 1 to 50 mg/mL solutions of the compounds of General Formulae (V) to (VIII), which were produced by introducing an amino group to the end of the hydrophilic polymer chain contained in General Formulae (I) to (IV). Condensation reaction was allowed to proceed in the presence of 0.1% carbodiimide, and the fabric was then rinsed with physiological saline, to provide an antithrombotic tubular fabric to be used as an artificial blood vessel.

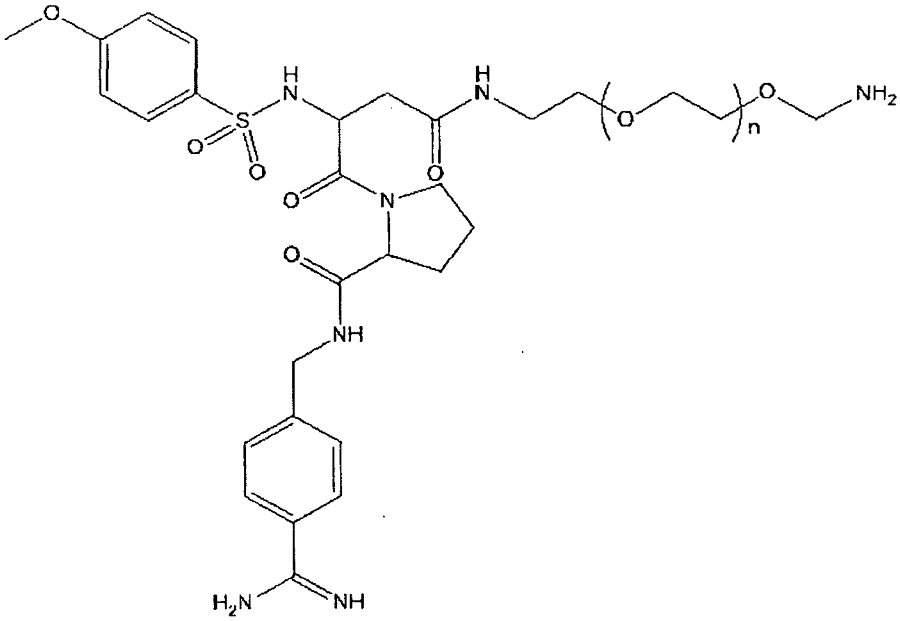

(V)

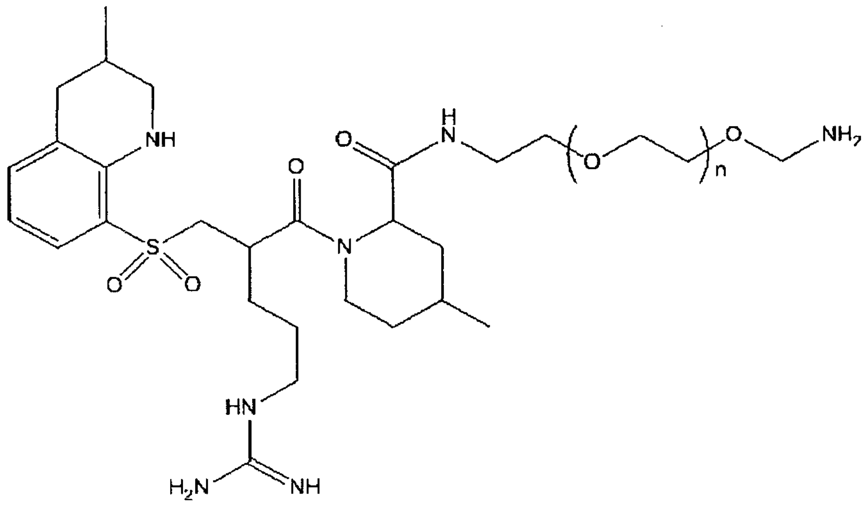

(VI)

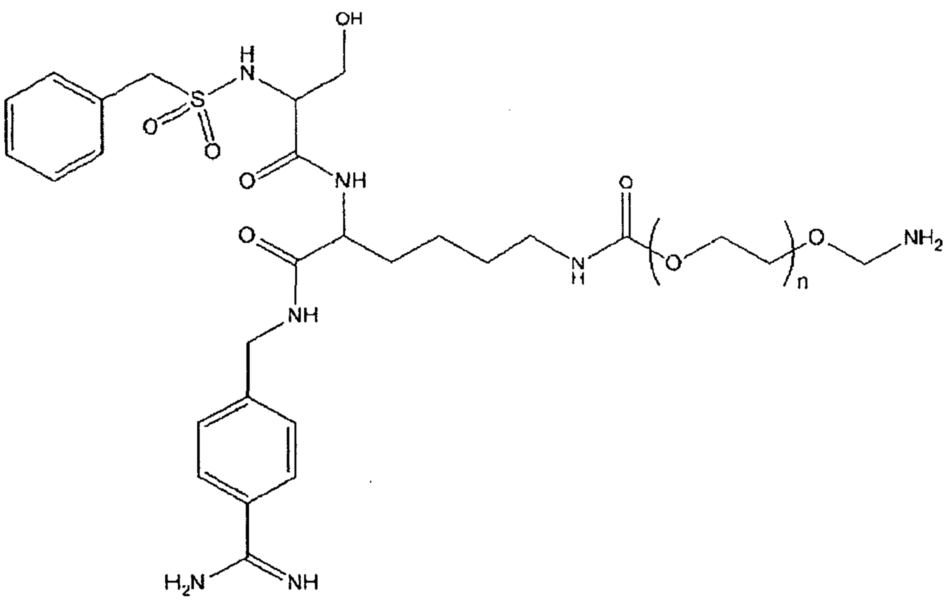

(VII)

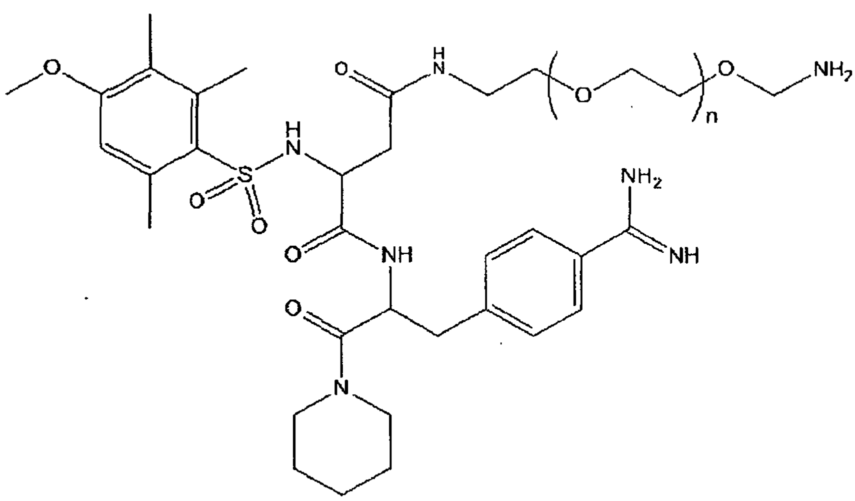

(VIII)

Table 1 shows the performance evaluation results of each antithrombotic tubular fabric, obtained by measurement of the water permeability, the thrombin activity inhibition rate in an extract, the thrombin activity inhibition rate on the fiber surface, the platelet adhesion rate, the cell adhesion rate, and thrombus adhesion.

Here, the antithrombotic tubular fabric treated in 1 mg/mL of the antithrombin agent of General Formula (V) was provided as Example 1; the antithrombotic tubular fabric treated in 2 mg/mL of the antithrombogenic agent of General Formula (V) was provided as Example 2; the antithrombotic tubular fabric treated in 5 mg/mL of the antithrombogenic agent of General Formula (V) was provided as Example 3; the antithrombotic tubular fabric treated in 10 mg/mL of the antithombogenic agent of General Formula (V) was provided as Example 4; the antithrombotic tubular fabric treated in 20 mg/mL of the antithrombogenic agent of General Formula (V) was provided as Example 5; the antithrombotic tubular fabric treated in 50 mg/mL of the antithrombogenic agent of General Formula (V) was provided as Example 6; the antithrombotic tubular fabric treated in 50 mg/mL of the antithrombogenic agent of General Formula (VI) was provided as Example 7; the antithrombotic tubular fabric treated in 50 mg/mL of the antithombogenic agent of General Formula (VII) was provided as Example 8; and the antithrombotic tubular fabric treated in 50 mg/mL of the antithombogenic agent of General Formula (VIII) was provided as Example 9.

(Comparative Examples)

The same tubular fabric as that obtained in Example 1 was immersed in 50 mg/mL aqueous solutions of the antithrombogenic agents of General Formulae (I) to (IV),
and, in this state, irradiated with γ-ray at 5 kGy at Koga Isotope. Each fabric was washed with Triton-X100 and water, to provide an antithrombotic tubular fabric.

Here, the antithrombotic tubular fabric irradiated with γ-ray in 50 mg/mL of the antithrombogenic agent of General Formula (I) was provided as Comparative Example 1; the antithrombotic tubular fabric irradiated with γ-ray in 50 mg/mL of the antithrombogenic agent of General Formula (II) was provided as Comparative Example 2; the antithrombotic tubular fabric irradiated with γ-ray in 50 mg/mL of the antithrombogenic agent of General Formula (III) was provided as Comparative Example 3; and the antithrombotic tubular fabric irradiated with γ-ray in 50 mg/mL of the antithrombogenic agent of General Formula (IV) was provided as Comparative Example 4. Table 1 shows the performance evaluation results of each antithrombotic tubular fabric, obtained by measurement of the water permeability, the thrombin activity inhibition rate in an extract, the thrombin activity inhibition rate on the fiber surface, the platelet adhesion rate, the cell adhesion rate, and thrombus adhesion.

A tubular fabric was prepared as a high-density plain-weave tissue using polyethylene terephthalate of 55 Dtex-48 f as a warp and polymer array fiber of 245 Dtex-40 f as a weft. The polymer array fiber used therefor was composed of 20 parts of polystyrene as sea-component and 80 parts of polyethylene terephthalate as island-component, and the number of islands was 36/f. This tubular fabric was sufficiently treated with hot water containing NaOH at 80°C, and immersed in toluene. Subsequently, the fabric was subjected to raising by using a raising machine, and then to water-jet punching. The fabric was then immersed in 50 mg/mL aqueous solution of the antithrombogenic agent of General Formula (I), and, in this state, irradiated with γ-ray at 5 kGy at Koga Isotope. The fabric was washed with Triton-X100 and water, to provide an antithrombotic tubular fabric (Comparative Example 5). Table 1 shows the performance evaluation results of the antithrombotic tubular fabric, obtained by measurement of the water permeability, the thrombin activity inhibition rate in an extract, the thrombin activity inhibition rate on the fiber surface, the platelet adhesion rate, the cell adhesion rate, and thrombus adhesion.

A tubular fabric was prepared as a low-density plain-weave tissue using polyethylene terephthalate of 55 Dtex-48 f as a warp and polymer array fiber of 245 Dtex-40 f as a weft. The polymer array fiber used therefor was composed of 20 parts of polystyrene as sea-component and 80 parts of polyethylene terephthalate as island-component, and the number of islands was 36/f. This tubular fabric was sufficiently treated with hot water containing NaOH at 80°C, and immersed in toluene. Subsequently, the fabric was subjected to raising by using a raising machine, and then to water-jet punching. The fabric was then immersed in 50 mg/mL aqueous solution of the antithrombogenic agent of General Formula (I), and, in this state, irradiated with γ-ray at 5 kGy at Koga Isotope. The fabric was washed with Triton-X100 and water, to provide an antithrombotic tubular fabric (Comparative Example 6). Table 1 shows the performance evaluation results of the antithrombotic tubular fabric, obtained by measurement of the water permeability, the thrombin activity inhibition rate in an extract, the thrombin activity inhibition rate on the fiber surface, the platelet adhesion rate, the cell adhesion rate, and thrombus adhesion.

A tubular fabric was prepared as a plain-weave tissue using polyethylene terephthalate of 55 Dtex-48 f both as a warp and as a weft. The fabric was immersed in 50 mg/mL aqueous solution of the antithrombogenic agent of General Formula (I), and, in this state, irradiated with γ-ray at 5 kGy at Koga Isotope. The fabric was washed with Triton-X100 and water, to provide an antithrombotic tubular fabric (Comparative Example 7). Table 1 shows the performance evaluation results of the antithrombotic tubular fabric, obtained by measurement of the water permeability, the thrombin activity inhibition rate in an extract, the thrombin activity inhibition rate on the fiber surface, the platelet adhesion rate, the cell adhesion rate, and thrombus adhesion.

To a solution of the compound of General Formula (IX) in dimethylformamide, 4 N hydrochloric acid/1,4-dioxane (Toyo Kasei Co., Ltd.) was added dropwise to allow the reaction to proceed, to obtain the hydrochloric acid salt of the General Formula (IX). To the solution of the hydrochloric acid salt in dimethylformamide, dicyclohexylcarbodiimide and 4-hydroxybenzotriazole were added, and polyether-modified silicone (X-22-3939A, Shin-Etsu Chemical Co., Ltd.) was further added thereto, followed by allowing the reaction to proceed. The resulting reaction liquid was then placed in a dialysis tube (Spectra/Por RC Por 6, MWCO=1000), and dialyzed against 10 volumes of distilled water. The reaction liquid after the dialysis was filtered, and the solvent in the filtrate was removed, followed by drying the resultant to obtain a hydrophilic polymer compound. The fabric was then immersed in 50 mg/mL aqueous solution of the hydrophilic polymer compound obtained, and, in this state, irradiated with γ-ray at 5 kGy at Koga Isotope. The fabric was washed with Triton-X100 and water, to provide an antithrombotic tubular fabric (Comparative Example 8). Table 1 shows the performance evaluation results of the antithrombotic tubular fabric, obtained by measurement of the water permeability, the thrombin activity inhibition rate in an extract, the thrombin activity inhibition rate on the fiber surface, the platelet adhesion rate, the cell adhesion rate, and thrombus adhesion.

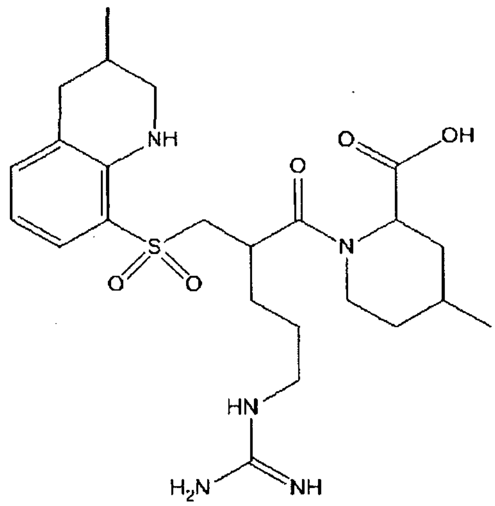

(IX)

[Table 1]

| Sample | | | | Anti-thrombogenic agent | Hydrophilic polymer | Water permeability ($mL/cm^2/min$) | Concentration of antithrombin activity substance used for treatment (mg/mL) | Thrombin activity inhibition rate in extract (%) | Thrombin activity inhibition rate on fiber surface (%) | Platelet adhesion rate (%) | Cell adhesion rate (%) | Thrombus adhesion |
|---|---|---|---|---|---|---|---|---|---|---|---|---|
| Example | 1 | PET fiber | Ultrafine fiber | V | PEG | 130 | 1 | 0.3 | 68 | 4 | 84 | - |
| | 2 | PET fiber | Ultrafine fiber | V | PEG | 1400 | 2 | 0.3 | 73 | 6 | 88 | - |
| | 3 | PET fiber | Ultrafine fiber | V | PEG | 2330 | 5 | 0.2 | 77 | 3 | 87 | - |
| | 4 | PET fiber | Ultrafine fiber | V | PEG | 3420 | 10 | 0.5 | 84 | 8 | 92 | - |
| | 5 | PET fiber | Ultrafine fiber | V | PEG | 2100 | 20 | 0.1 | 89 | 8 | 83 | - |
| | 6 | PET fiber | Ultrafine fiber | V | PEG | 2080 | 50 | 0.2 | 90 | 8 | 91 | - |
| | 7 | PET fiber | Ultrafine fiber | VI | PEG | 2410 | 50 | 0.9 | 93 | 6 | 94 | - |
| | 8 | PET fiber | Ultrafine fiber | VII | PEG | 2250 | 50 | 1.2 | 90 | 1 | 89 | - |
| | 9 | PET fiber | Ultrafine fiber | VIII | PEG | 2160 | 50 | 0.6 | 87 | 4 | 90 | - |
| Comparative Example | 1 | PET fiber | Ultrafine fiber | I | - | 3390 | 50 | 0.6 | 56 | 14 | 83 | + |
| | 2 | PET fiber | Ultrafine fiber | II | - | 2490 | 50 | 0.3 | 41 | 7 | 72 | + |
| | 3 | PET fiber | Ultrafine fiber | III | - | 2250 | 50 | 0.4 | 43 | 11 | 86 | + |
| | 4 | PET fiber | Ultrafine fiber | IV | - | 2730 | 50 | 0.3 | 38 | 10 | 81 | + |
| | 5 | PET fiber | Ultrafine fiber | I | - | 50 | 50 | 0.2 | 30 | 16 | 54 | - |
| | 6 | PET fiber | Ultrafine fiber | I | - | 4380 | 50 | 0.2 | 52 | 28 | 34 | Blood leakage |
| | 7 | PET fiber | - | I | - | 4610 | 50 | 0.2 | 35 | 21 | 12 | Blood leakage |
| | 8 | PET fiber | Ultrafine fiber | I | - | 2510 | 50 | 0.2 | 31 | 21 | 75 | - |

As shown in Table 1, in the cases where an antithrombogenic agent was immobilized by condensation reaction, the antithrombin activity on the fiber surface of the artificial blood vessel was high, and no thrombus formation occurred during the circulation. On the other hand, in the cases where an antithrombogenic agent was immobilized by $\gamma$-ray irradiation, the surface antithrombin activity was low, and thrombus formation occurred. In the cases where the water permeability was high, blood leakage occurred during the circulation. Also in the cases where the immobilization was carried out in the absence of a spacer, thrombus formation occurred. In cases where an antithrombogenic agent is immobilized by condensation reaction, the surface treatment can be achieved also in deep portions of the microstructure of the fiber, so that the effect to suppress thrombus formation is high.

INDUSTRIAL APPLICABILITY

The present invention can be used as an artificial blood vessel with which both antithrombogenicity and cellular affinity can be achieved, which promotes intimal formation after indwelling and maintains antithrombogenicity during the intimal formation, and which can maintain its patency for a long time.

DESCRIPTION OF SYMBOLS

1...Ultrafine fiber, 2...Fiber layer, 3...Ultrafine fiber layer

## Claims

1. An artificial blood vessel which is a tubular fabric comprising, a fiber layer containing an ultrafine fiber(s) and an ultrafine fiber layer in the inside of the fiber layer, the ultrafine fiber layer being composed of an ultrafine fiber(s) having a fiber diameter(s) of not less than 10 nm and not more than 3 $\mu$m, wherein
an antithrombogenic agent having a polymer chain other than heparin is covalently bound to said ultrafine fiber via said polymer chain by a condensation reaction, an addition reaction, or graft polymerisation, and
the thrombin activity inhibition rate on the fiber surface at 37°C is not less than 60%.

2. The artificial blood vessel according to claim 1, wherein the molecular weight of said antithrombogenic agent is not more than 3000.

3. The artificial blood vessel according to claim 1 or 2, whose water permeability at 120 mmHg is not less than 100 $mL/cm^2/min$ and less than 4000 $mL/cm^2/min$.

4. The artificial blood vessel according to any one of claims 1 to 3, wherein the thrombin activity inhibition rate in an extract obtained by 24 hours of extraction at 37°C in 10 mL of physiological saline per 1 g of said artificial blood vessel is less than 5%.

5. The artificial blood vessel according to any one of claims 1 to 4, wherein said antithrombogenic agent has a guanidino group, guanido group, and/or amidino group

6. The artificial blood vessel according to any one of claims 1 to 5, wherein said polymer chain is a polymer structure selected from polyalkylene glycol, polyvinyl alcohol, and polyvinyl pyrrolidone.

7. The artificial blood vessel according to any one of claims 1 to 6, wherein said antithrombogenic agent is selected from the following Chemical Formulae (I) to (IV):

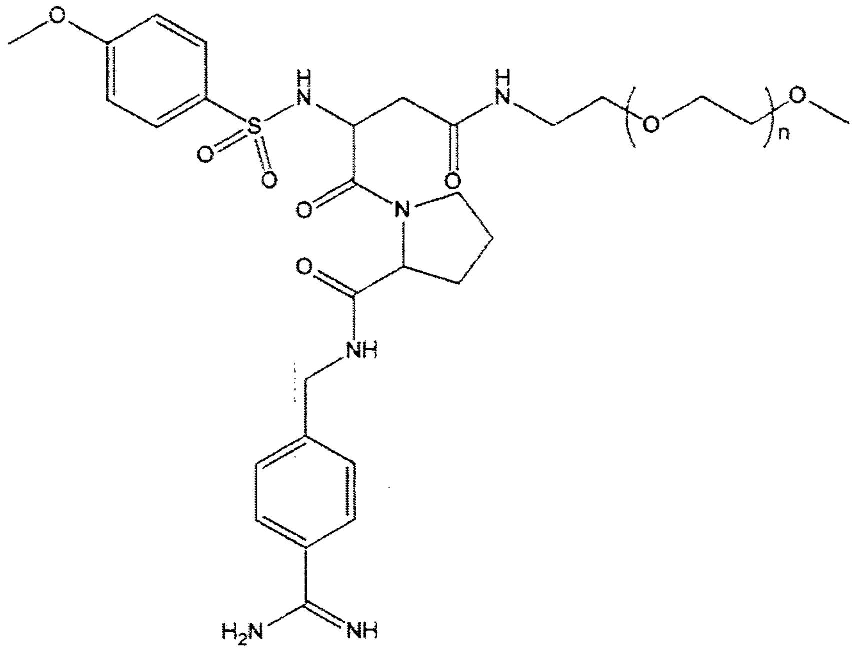

(I);

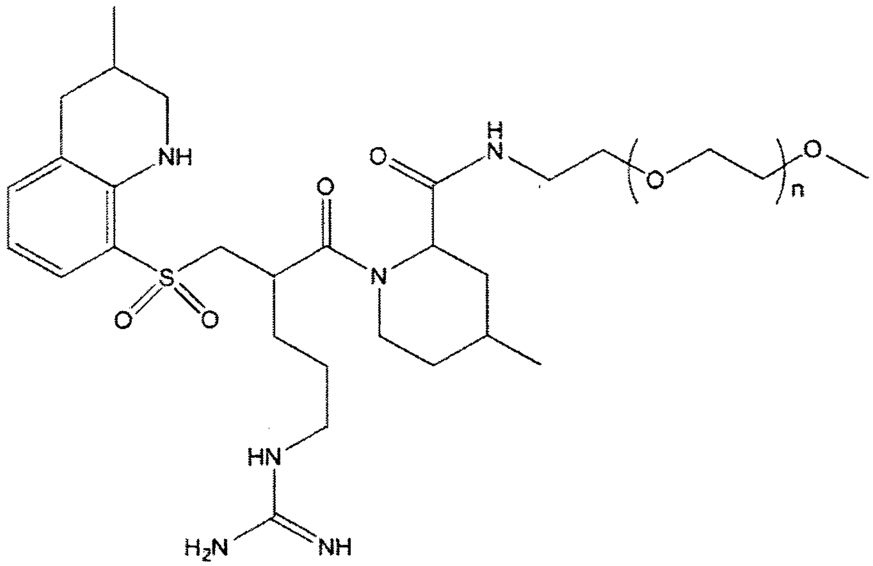

(II);

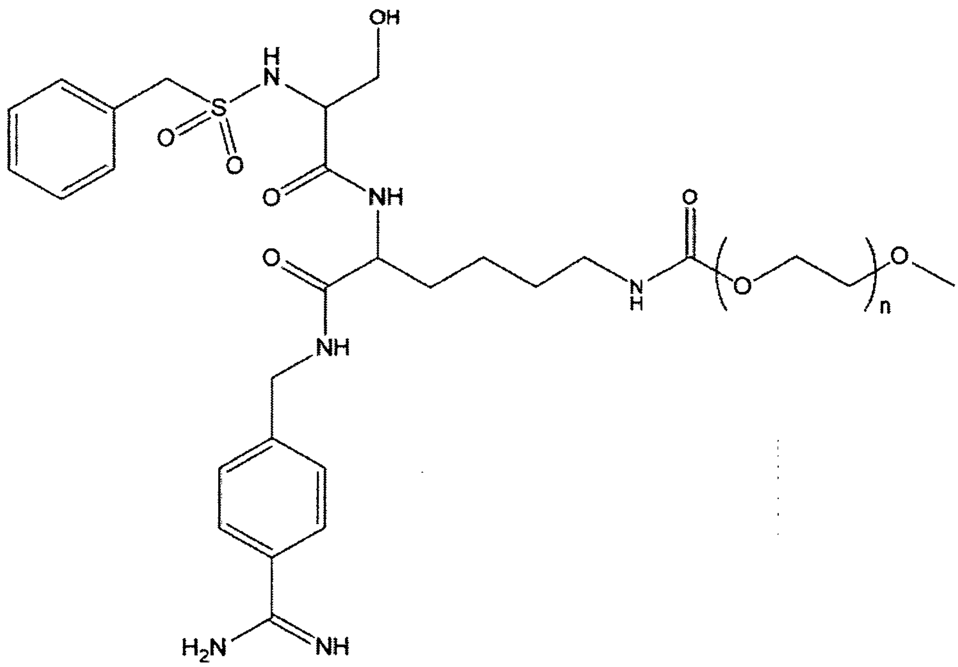

(III);

and

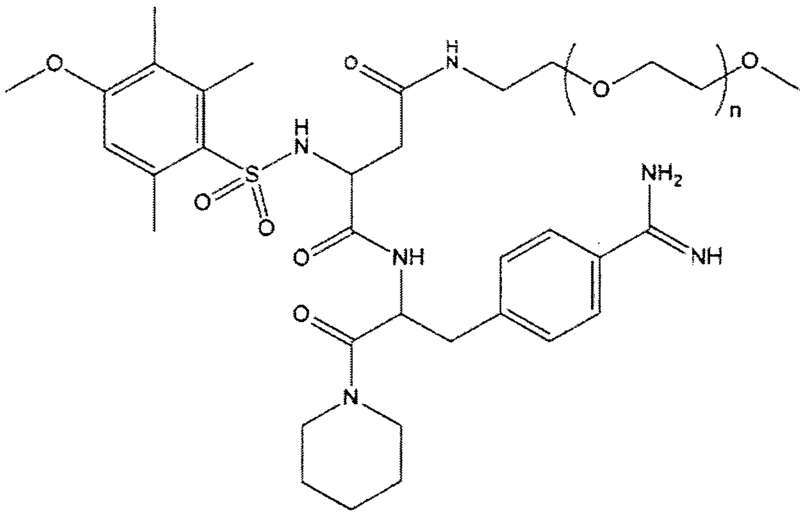

(IV);

(wherein n represents an integer of 1 to 500).

**8.** The artificial blood vessel according to any one of claims 1 to 7, wherein said fiber layer is composed of said ultrafine fiber(s) and a multifilament(s), the multifilament(s) having a total fineness of 1 to 60 decitex.

**9.** The artificial blood vessel according to claim 8, wherein the fineness of single yarns constituting said multifilament is 0.5 to 10.0 decitex.

**10.** The artificial blood vessel according to any one of claims 1 to 9, having a platelet adhesion rate of less than 20%.

**11.** The artificial blood vessel according to any one of claims 1 to 10, wherein said tubular fabric is composed of a polyester fiber(s).

**12.** The artificial blood vessel according to any one of claims 1 to 11, wherein the inner diameter of said tubular fabric is not less than 1 mm and less than 10 mm.

**Patentansprüche**

1. Künstliches Blutgefäß, das ein schlauchförmiges Gewebe ist, umfassend eine Faserschicht, die eine Ultrafeinfaser(n) enthält, und eine Ultrafeinfaserschicht in der Innenseite der Faserschicht, wobei die Ultrafeinfaserschicht aus einer Ultrafeinfaser(n) besteht, die einen Faserdurchmesser (die Faserdurchmesser) von nicht weniger als 10 nm und nicht mehr als 3 μm aufweist(en), wobei
ein antithrombotisches Mittel, das eine Polymerkette mit Ausnahme der von Heparin aufweist, das über die Polymerkette mittels einer Kondensationsreaktion, einer Additionsreaktion oder Pfropfpolymerisation kovalent an die Ultrafeinfaser gebunden ist, und
die Thrombinaktivitätsinhibitionsrate bei 37 °C auf der Faseroberfläche nicht weniger als 60 % beträgt.

2. Künstliches Blutgefäß nach Anspruch 1, wobei das Molekulargewicht von dem antithrombotischen Mittel nicht mehr als 3000 beträgt.

3. Künstliches Blutgefäß nach Anspruch 1 oder 2, dessen Wasserpermeabilität bei 120 mmHg nicht weniger als 100 $ml/cm^2/min$ und weniger als 4000 $ml/cm^2/min$ beträgt.

4. Künstliches Blutgefäß nach einem der Ansprüche 1 bis 3, wobei die Thrombinaktivitätsinhibitionsrate in einem Extrakt, der durch eine 24-stündige Extraktion bei 37 °C in 10 ml physiologischer Kochsalzlösung pro 1 g des künstlichen Blutgefäßes erhalten wird, weniger als 5 % beträgt.

5. Künstliches Blutgefäß nach einem der Ansprüche 1 bis 4, wobei das antithrombotische Mittel eine Guanidinogruppe, Guanidogruppe und/oder Amidinogruppe aufweist.

6. Künstliches Blutgefäß nach einem der Ansprüche 1 bis 5, wobei die Polymerkette eine Polymerstruktur ist, welche aus Polyalkylenglycol, Polyvinylalkohol und Polyvinylpyrrolidon ausgewählt ist.

7. Künstliches Blutgefäß nach einem der Ansprüche 1 bis 6, wobei das antithrombotische Mittel aus den folgenden chemischen Formeln (I) bis (IV) ausgewählt ist:

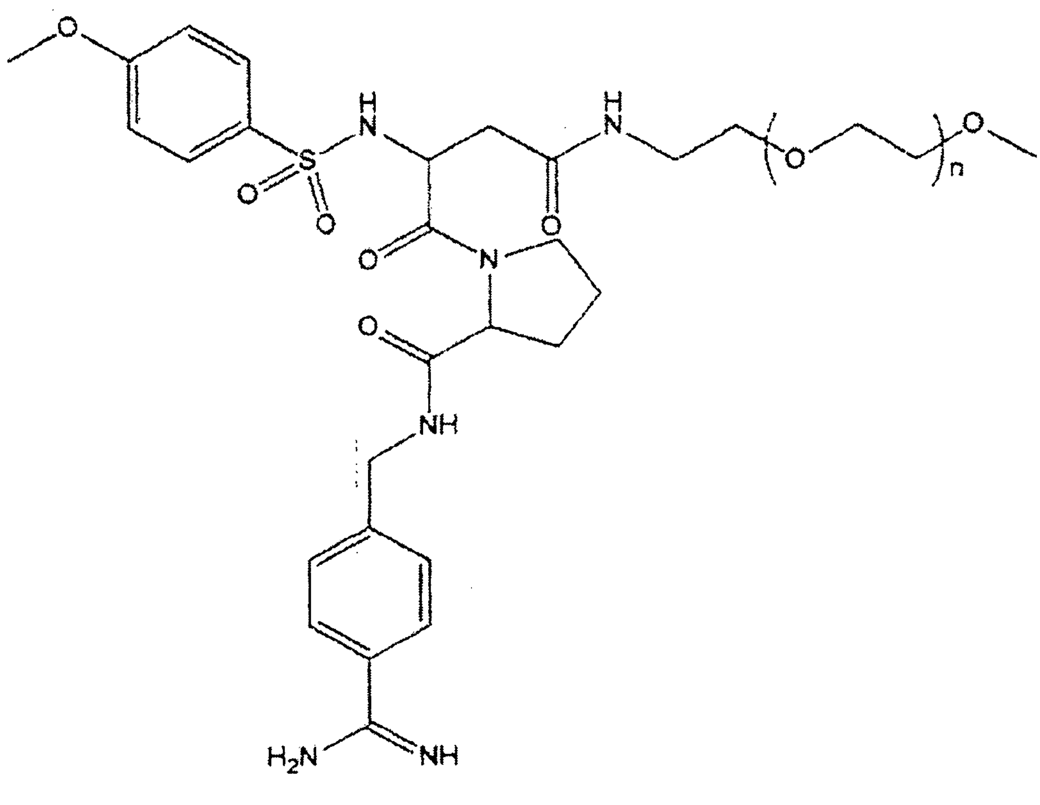

(I);

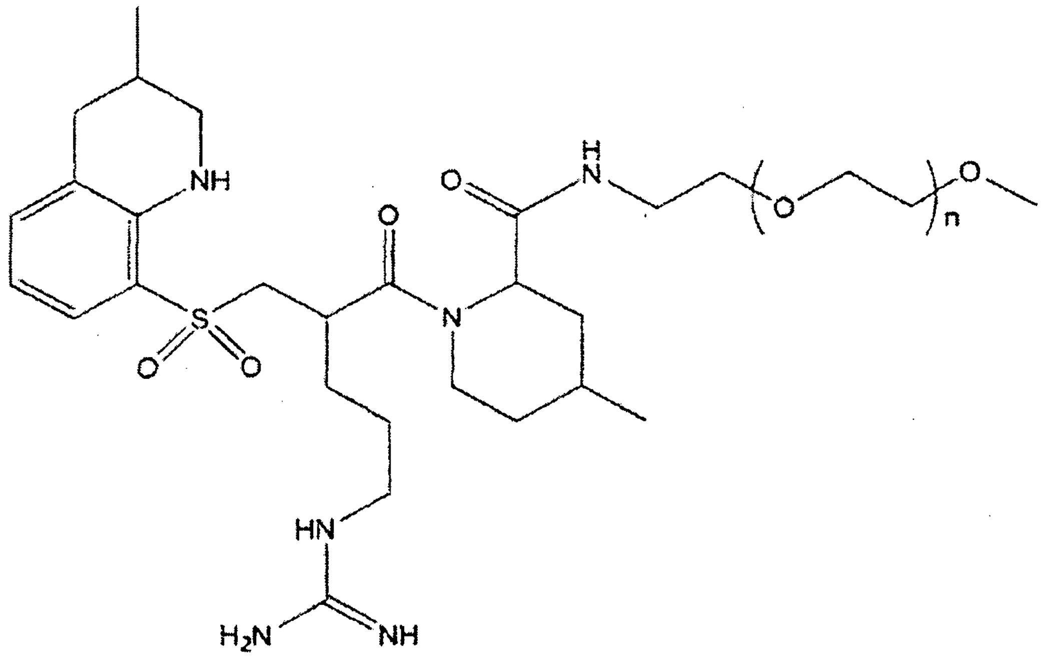

(II);

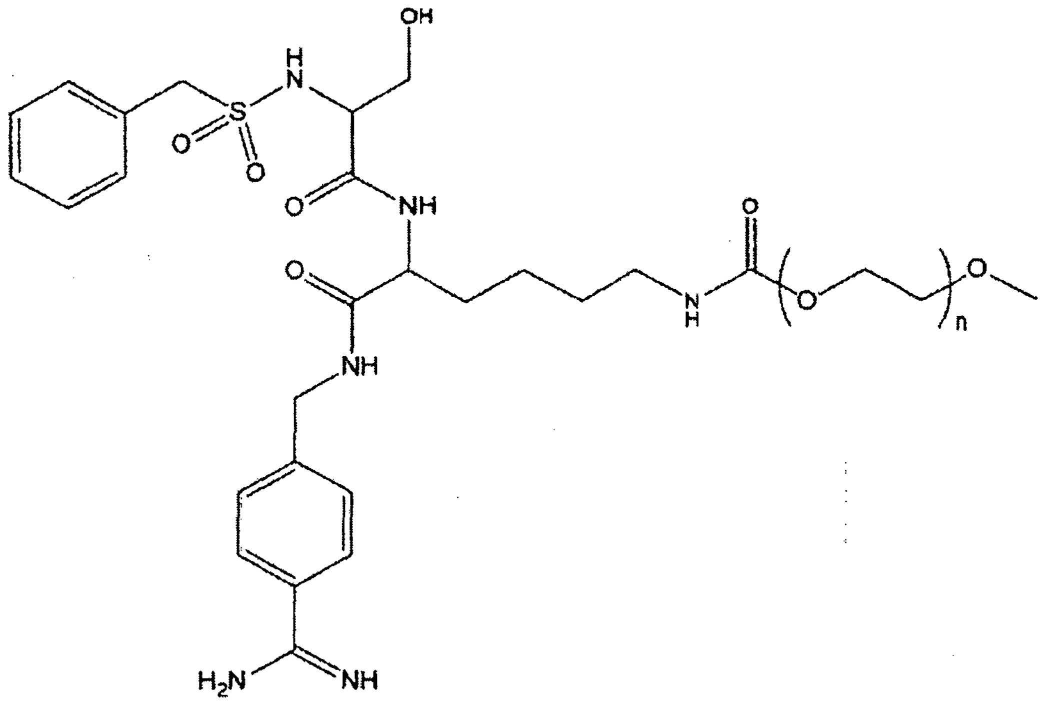

(III);

und

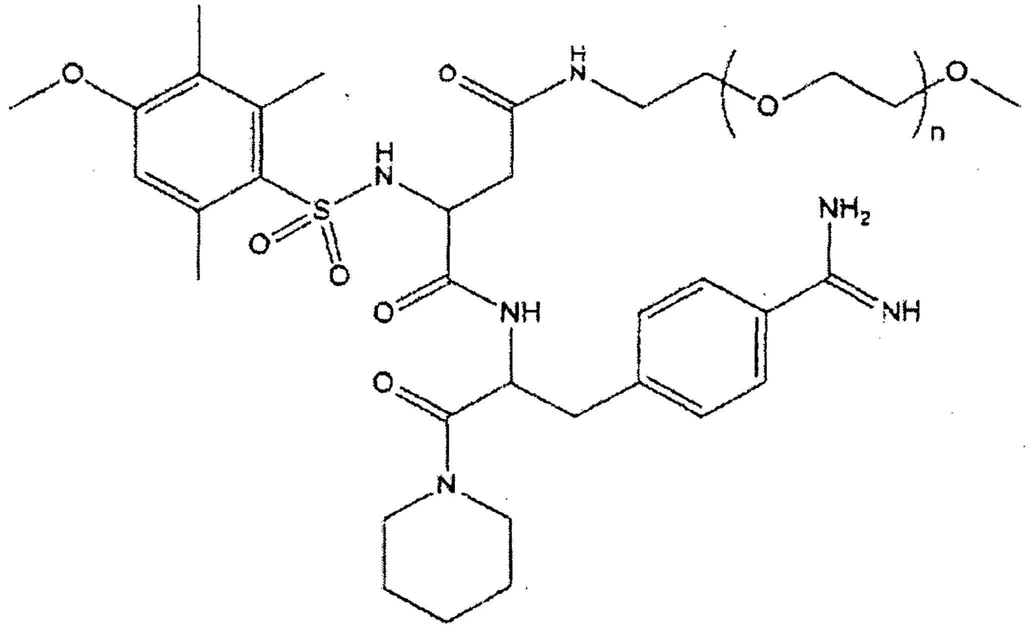

(IV);

(wobei n eine ganze Zahl von 1 bis 500 repräsentiert).

8. Künstliches Blutgefäß nach einem der Ansprüche 1 bis 7, wobei die Faserschicht aus der (den) Ultrafeinfaser(n) und einem Multfilament(en) besteht, wobei das (die) Multifilament(e) eine Gesamtfeinheit von 1 bis 60 Decitex aufweist (aufweisen).

9. Künstliches Blutgefäß nach Anspruch 8, wobei die Feinheit von einzelnen Garnen, die das Multifilament ausmachen, 0,5 bis 10,0 Decitex beträgt.

10. Künstliches Blutgefäß nach einem der Ansprüche 1 bis 9, das eine Thrombozytenadhäsivitätsrate von weniger als 20 % aufweist.

11. Künstliches Blutgefäß nach einem der Ansprüche 1 bis 10, wobei das schlauchförmige Gewebe aus einer Polyesterfaser(n) besteht.

12. Künstliches Blutgefäß nach einem der Ansprüche 1 bis 11, wobei der Innendurchmesser des schlauchförmigen Gewebes nicht weniger als 1 mm und weniger als 10 mm beträgt.

**Revendications**

1. Vaisseau sanguin artificiel qui est un tissu tubulaire comprenant, une couche de fibres contenant une ou plusieurs fibre(s) ultrafine(s) et une couche de fibres ultrafines à l'intérieur de la couche de fibres, la couche de fibres ultrafines étant composée d'une ou plusieurs fibre(s) ultrafine(s) ayant un ou plusieurs diamètre(s) de fibre d'au moins 10 nm et de pas plus de 3 μm, dans lequel
un agent antithrombogène ayant une chaîne de polymère autre que l'héparine est lié de façon covalente à ladite fibre ultrafine par l'intermédiaire de ladite chaîne de polymère par une réaction de condensation, une réaction d'addition ou une polymérisation par greffage, et
le taux d'inhibition de l'activité thrombine sur la surface des fibres à 37 °C n'est pas inférieur à 60 %.

2. Vaisseau sanguin artificiel selon la revendication 1, dans lequel le poids moléculaire dudit agent antithrombogène n'est pas supérieur à 3000.

3. Vaisseau sanguin artificiel selon la revendication 1 ou 2, dont la perméabilité à l'eau à 120 mmHg n'est pas inférieure à 100 ml/cm$^2$/min et est inférieure à 4000 ml/cm$^2$/min.

4. Vaisseau sanguin artificiel selon l'une quelconque des revendications 1 à 3, dans lequel le taux d'inhibition de l'activité thrombine dans un extrait obtenu par 24 heures d'extraction à 37 °C dans 10 ml de sérum physiologique par 1 g dudit vaisseau sanguin artificiel est inférieur à 5 %.

5. Vaisseau sanguin artificiel selon l'une quelconque des revendications 1 à 4, dans lequel ledit agent antithrombogène comporte un groupe guanidino, un groupe guanido et/ou un groupe amidino.

6. Vaisseau sanguin artificiel selon l'une quelconque des revendications 1 à 5, dans lequel ladite chaîne de polymère est une structure de polymère choisie parmi le polyalkylène glycol, l'alcool polyvinylique et la polyvinylpyrrolidone.

7. Vaisseau sanguin artificiel selon l'une quelconque des revendications 1 à 6, dans lequel ledit agent antithrombogène est choisi parmi les formules chimiques (I) à (IV) suivantes :

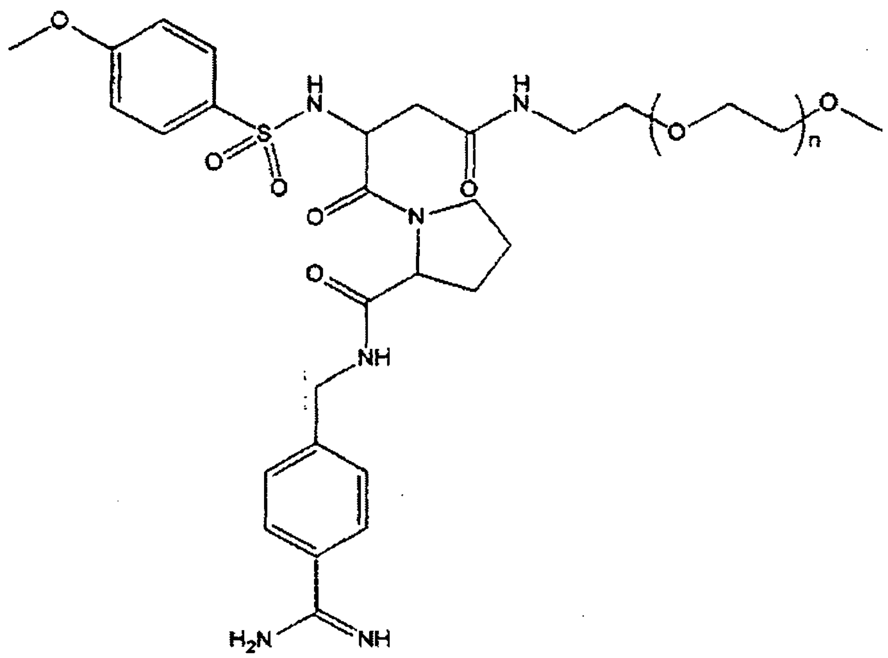

(I);

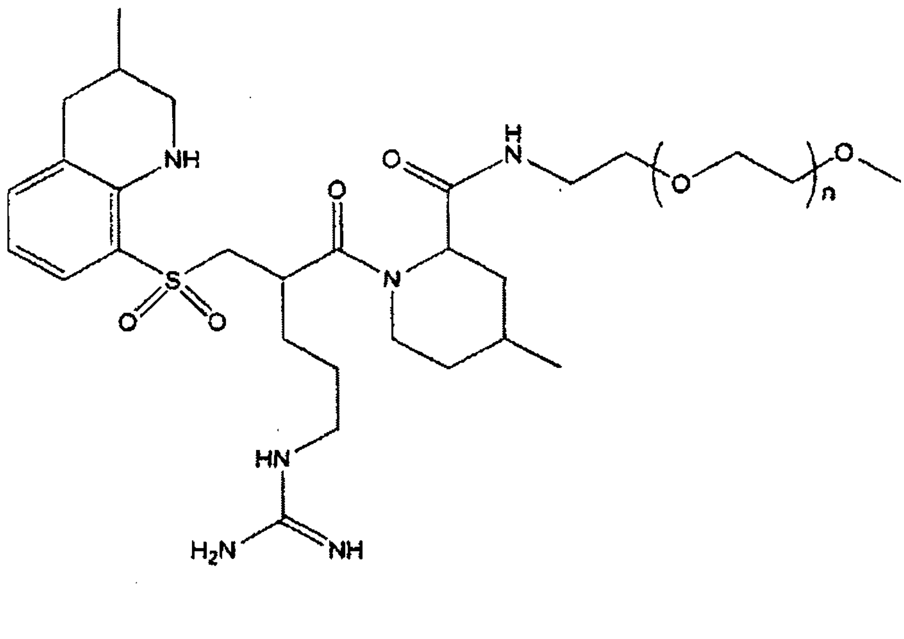

(II);

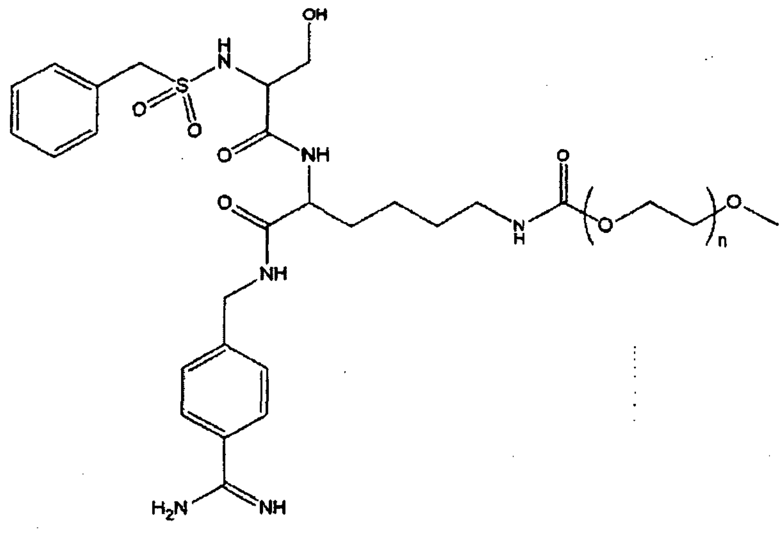

(III) ;

et

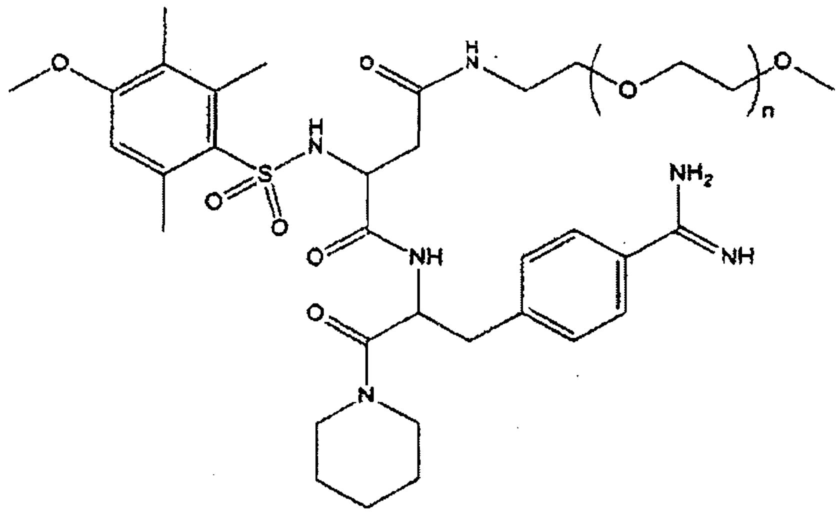

(IV) ;

(dans lesquelles n représente un entier de 1 à 500).

8. Vaisseau sanguin artificiel selon l'une quelconque des revendications 1 à 7, dans lequel ladite couche de fibres est composée desdites une ou plusieurs fibre(s) ultrafine(s) et d'un ou plusieurs multifilament(s), le(s) multifilament(s) ayant une finesse totale de 1 à 60 decitex.

9. Vaisseau sanguin artificiel selon la revendication 8, dans lequel la finesse de simples fils constituant ledit multifilament est de 0,5 à 10,0 decitex.

10. Vaisseau sanguin artificiel selon l'une quelconque des revendications 1 à 9, ayant un taux d'adhésion plaquettaire inférieur à 20 %.

11. Vaisseau sanguin artificiel selon l'une quelconque des revendications 1 à 10, dans lequel ledit tissu tubulaire est composé de fibre(s) de polyester.

12. Vaisseau sanguin artificiel selon l'une quelconque des revendications 1 à 11, dans lequel le diamètre interne dudit tissu tubulaire n'est pas inférieur à 1 mm et est inférieur à 10 mm.

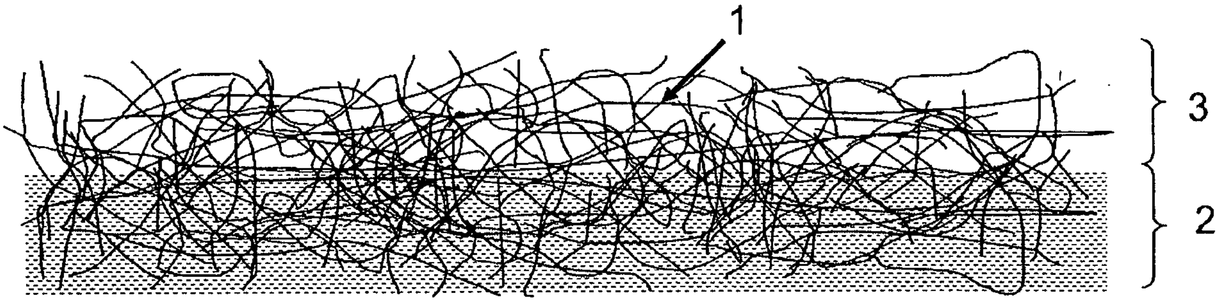


Fig.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 1875991 B
- JP 1870688 B
- JP 1338011 B
- JP 4627978 B
- JP 3799626 B
- JP 2009545333 W
- JP 2014061408 B
- WO 08032758 A
- WO 2011078208 A